(19) Europäisches Patentamt
European Patent Office
Office européen des brevets

(11) **EP 2 944 698 B1**

(12) **EUROPEAN PATENT SPECIFICATION**

(45) Date of publication and mention of the grant of the patent:
**03.10.2018 Bulletin 2018/40**

(21) Application number: **14737982.0**

(22) Date of filing: **02.01.2014**

(51) Int Cl.:
*G01N 33/50* (2006.01)     *G01N 33/574* (2006.01)
*G01N 33/94* (2006.01)

(86) International application number:
**PCT/CN2014/000001**

(87) International publication number:
**WO 2014/108042 (17.07.2014 Gazette 2014/29)**

(54) **STERILE AND INSTANTLY DISSOLVABLE DRUG MEMBRANE AND USE THEREOF FOR TESTING TUMOR DRUG SENSITIVITY**

STERILE UND SOFORT LÖSLICHE WIRKSTOFFMEMBRAN UND VERWENDUNG ZUM TESTEN DER SENSITIVITÄT VON TUMORWIRKSTOFFEN

FILM À DISSOLUTION RAPIDE EXEMPT DE BACTÉRIES ET SES APPLICATIONS DANS LE CADRE DE TESTS DE SENSIBILITÉ AUX MÉDICAMENTS ANTITUMORAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.01.2013 CN 201310006751**

(43) Date of publication of application:
**18.11.2015 Bulletin 2015/47**

(73) Proprietor: **Suzhou Macwell Biomedical Sci & Tech Co. Ltd**
**Jiangsu 215123 (CN)**

(72) Inventors:
• **WANG, Shouli**
**Suzhou**
**Jiangsu 215123 (CN)**
• **QI, Wenrong**
**Suzhou**
**Jiangsu 215123 (CN)**
• **SONG, Renbo**
**Suzhou**
**Jiangsu 215123 (CN)**
• **TAO, Zhengyu**
**Suzhou**
**Jiangsu 215123 (CN)**
• **DENG, Gang**
**Suzhou**
**Jiangsu 215123 (CN)**

• **QIU, Ruibao**
**Suzhou**
**Jiangsu 215123 (CN)**
• **WU, Minghui**
**Suzhou**
**Jiangsu 215123 (CN)**

(74) Representative: **Petho, Arpad**
**Danubia**
**Patent & Law Office LLC**
**POB 198**
**1368 Budapest (HU)**

(56) References cited:
**CN-A- 102 018 686     CN-A- 102 018 686**
**CN-A- 103 088 102     CN-Y- 201 195 724**

• **Apoorva Mahajan: "Formulation & Evaluation of Fast dissolving Buccal films of Sertraline", International Journal of Drug Development & Research, 1 March 2012 (2012-03-01), pages 220-226, XP055282723, Retrieved from the Internet: URL:http://www.ijddr.in/drug-development/f ormulation--evaluation-of-fast-dissolving- buccal-films-ofsertraline.pdf [retrieved on 2016-06-27]**

**(Cont. next page)**

- Nishi Thakur ET AL: "Overview "A Novel Approach of Fast Dissolving Films and Their Patients"", Advances in Biological Research, 1 January 2013 (2013-01-01), pages 50-58, XP055282727, DOI: 10.5829/idosi.abr.2013.7.2.72134 Retrieved from the Internet: URL:http://idosi.org/abr/7(2)13/4.pdf [retrieved on 2016-06-27]
- ZHANG ET AL: "The study of Controlled Release Dosage Form of Antineoplastic VCR", SYMPOSIUM OF THE 4TH MEETING OF THE CHINESE DOCTOR'S FORUM ON NEW MEDICINE; 01/11/1999; BEIJING, CHINA, ZHONGHUÁ ZHONG YIY GBP AO XUÉHU GBP I TÁNGNI GBP AOB GBP ING FENHU GBP I, CN, 1 January 1999 (1999-01-01), pages 262-268, XP008179729,
- ZHANG, QIQING ET AL.: 'The study of Controlled Release Dosage Form of Antineoplastic VCR.' PROCEEDINGS OF 4TH DOCTOR FORUM ON NEW MEDICINE IN CHINA 1999, pages 262 - 268, XP008179729
- TAN, YUNPU ET AL.: '5-FU Loaded Nanofi bers Membrane for Controlled Release Effect Against Colon Cancer Cell in Vitro.' CHIN J BASES CLIN GENERAL SURG vol. 19, no. 6, 2012, pages 610 - 615, XP008179730

**Description**

BACKGROUND OF THE PRESENT INVENTION

**Field of Invention**

The present invention relates to a method for producing a sterile and instantly dissolvable drug membrane, a method for testing drug sensitivity of antineoplastic drugs using membranes produced by said method, and a use of said membranes for testing drug sensitivity of antineoplastic drugs, which belong to a field of medical technology.

**Description of Related Arts**

Cancer is a common disease which seriously harms human life and health, and is a major cause of disability and premature death. For an age group from 35 to 59, cancer ranks a first cause of death. Statistics show that the number of cancer cases in China is annually 2 million, wherein 1.5 million thereof end up with death. Moreover, the number increases by 3% per year and patients tend to be younger. Cancer is the most deadly in all kinds of diseases.
Conventionally, main treatment methods for cancer comprise surgery, radiotherapy, chemotherapy, hormonal therapy and immune therapy, wherein compared with other methods, chemotherapy, as a systemic treatment, is able to kill tumor cells as many as possible. Therefore, chemotherapy plays a very important role in the treatment of cancer. With the development of medicine, chemotherapy is no longer simply a method of palliative treatment, and is changing from palliative to curative.
In 1998, the World Health Organization announced that appropriate chemotherapy had become a curing method for some tumors (such as malignant trophoblastic tumor, acute lymphocytic leukemia, Hodgkin's lymphoma, non-Hodgkin's lymphoma, testicular cancer, acute myeloid leukemia, embryonal rhabdomyosarcoma, skin cancer, small-cell lung cancer and ovarian cancer) has become the radical treatment can cure cancer. With the help of chemotherapy, tumors such as mammary cancer, osteogenic sarcoma, colon cancer, osteosarcoma, retinoblastoma, soft tissue sarcoma and renal blastoma are curable.
Although chemotherapy plays a very important role in the treatment of cancer, the results are often unsatisfactory in clinical practice. Tumor cells will become resistant to chemotherapy drugs, which is a common factor leading to failure of cancer chemotherapy, and is a key problem troubling cancer treatment. Drug resistance is a very common clinical problem. According to the American Cancer Society, more than 90% of patients died due to tumor were affected by drug resistance by varying degrees.
Tumor cell drug resistance is divided into primary resistance and acquired resistance. Conventional, clinical practice is generally based on evidence-based research results of international tumor clinical experiments, for obtaining that different chemotherapeutic drugs have different sensitivity to different tumor treatments, which means that each tumor has a corresponding effective chemotherapeutic drug sensitivity profile. Accordingly, a drug list with highest efficacy or pharmaceutical composition treatment is selected for treatment. However, in clinical, an effective treatment recognized by the evidence-based cancer research for certain tumor has no effect on some patients. For example, doxorubicin for invasive breast cancer is a landmark of treatment, but there are still 50% of invasive breast cancer patients who are not sensitive to the drug. Again, Gemzar is significantly effective for non-small cell lung cancer, but there are more than 60% of the patients receive no obvious effect from Gemzar.
Tumors have significant individual differences for various chemotherapeutic drugs. That is to say, for patients of different tumor types or different patients of the same type, or even the same patient at different stages, the sensitivity to chemotherapy is quite different, and treatment effects varies widely. So far there is not a chemotherapy drug or combination of several chemotherapy drugs, is 100% effective for a particular tumor. Clinically, it is apparently unreasonable to use the same chemotherapy drug or chemotherapy method on different tumor patients. Therefore, it is quite necessary to provide tumor chemotherapy sensitivity test on different patients for selecting effective drugs. For the propose, testing similar to bacterial sensitivity testing is necessary, for accurately screening chemotherapy drug according to different patients and determining the dosage with a reliable method, so as to truly personalize medicine clinic.
Conventionally, drugs for tumor drug sensitivity testing are mainly prepared right before utilization. Tumor drug sensitivity test needs to test a variety of antineoplastic drugs. Drug liquid is complex to prepare and dilute, and a dosage required is small, which is inconvenient to sample. Some of the drugs in a solution state are only able to be stored for a short time, and the remaining part must be wasted, resulting in waste of drugs, especially for expensive drugs.
The prior art discloses a tumor drug sensitivity testing methods, mainly using reagent kits. For example, Chinese patent CN93111551.5 discloses a tumor chemotherapy drug sensitivity prediction method and reagent kits thereof. According to the patent, 8 kinds of commonly used antineoplastic drugs are pre-set in specimen bottles with pre-determined dosages. However, the method is not suitable for testing combined chemotherapy, and is not suitable for adjusting dosages thereof.

Chinese patent CN03102260.X uses a pre-prepared drug sensitivity testing plate, adding antineoplastic drugs onto the plate with a pre-calculated dosage, freeze-dries and packages for cancer drug sensitivity testing. The method is also not suitable for testing combined chemotherapy, and is not suitable for adjusting dosages thereof. Furthermore, the method is not really sterile, which does not meet the requirements of sterile cell culture. Because the dosage of the drug added onto the plates is small, intersect dust pollution of different drugs will happen during freeze-drying. Therefore, the actual diagnosis is suspicious.

Apoorva Mahajan: "Formulation & Evaluation of Fast dissolving Buccal films of Sertraline", International Journal of Drug Development & Research, 1 March 2012 (2012-03-01), pages 220-226, XP055282723 (URL:http://www.ijddr.in/drug-development/formulation-evaluation-of-fast-dissolving-buccal-films-ofsertraline.pdf) discloses the formulation and evaluation of fast dissolving buccal films of sertraline, a selective serotonin reuptake inhibitor (SSRI). It also mentions formulations containing PVP as polymer (Table 2, e.g. formulation FA1). Said film is submitted to be fast dissolvable within 1 hour (i.e. it is instantly dissolvable) and suitable for sterilization. This publication, however, does not disclose the use of anti-cancer agents as active ingredient in this film or screening assays to test anti-cancer activity of the drugs.

Nishi Thakur ET AL: "Overview "A Novel Approach of Fast Dissolving Films and Their Patients"", Advances in Biological Research, 1 January 2013 (2013-01-01), pages 50-58, XP055282727, DOI: 10.5829/idosi.abr.2013.7.2.72134 (URL:http://idosi.org/abr/7(2)13/4.pdf) D2 discloses fast dissolving films. A typical composition for such film is shown in the last table of page 51. However, this publication does not disclose the use of anti-cancer drugs in such fast dissolving films, neither corresponding production methods or screening methods for testing anti-cancer drugs employing said films are described, nor production thereof.

ZHANG, QIQING ET AL: "The study of Controlled Release Dosage Form of Antineoplastic VCR.", PROCEEDINGS OF 4TH DOCTOR FORUM ON NEW MEDICINE IN CHINA, 1999, pages 262-268, XP008179729, discloses a method of carrying out a drug sensitivity test of an antineoplastic drug, comprising adding a release solution (which should be sterilized by filtration before being added) of a drug film in a culture solution to K562 tumour cells and detecting the inhibiting effect of the drug film on the in vitro culture of K562 tumour cells through the MTT method. This publication, however, does not disclose that the membrane would be "instantly dissolvable".

CN 102 018 686 A (SHAO YIFU HOSP ZHEJIANG UNI SCHOOL MED) 20 April 2011 (2011-04-20) discloses a method for preparing a film comprising mitomycin. This publication, however, does not disclose steps of degassing using heating, staying or ultrasound and, also, does not disclose any screening method of testing the anti-cancer activity of said drug film.

CN 201 195 724 Y 18 February 2009 (2009-02-18) relates to drug sensitivity testing of tumor cells. It uses a "checkout console" for this comprising a plate lid, a detection plate body with a plurality of detection holes and a storage medicine plate (see figure 1). In more detail, this publication discloses a tumor cell drug-sensitivity testing detection plate which comprises a plate cover (1) and a detection plate body (3), and a number of drug-sensitivity detection holes (31) are formed in the detection plate body (3). A drug storage plate (2) is further arranged between the detection plate body (3) and the plate cover (1). Corresponding to the drug-sensitive detection hole (31) in the detection plate body (3), a drug feeding hole (21) is formed in the drug storage plate (2); the drug feeding hole (21) is provided with a medicine film; the drug storage plate (2) covers the detection plate body (3), and the plate cover (1) covers the drug storage plate (2) (see claim 1 in D5). The drug feeding hole (21) in the drug storage plate (2) has a funnel shape and a small spoon (22) containing the medicine film is arranged in the middle of the bottom of the drug feeding hole (21) (see claim 2 in D5). Even though a "medicine film" is mentioned, no pre-produced drug membrane preparation is used in this publication. The drug to be tested is introduced into the small spoons (22) comprised in the drug feeding holes (21) in liquid solution form and is then freeze dried onto the surface of the spoons and this is how said "medicine film" is made.

Clinically, there are a few antineoplastic drug sensitivity testing methods whose antineoplastic drugs are prepared right before utilization. However, the methods have many problems: the exact concentration of the drug is unknown, the accuracy of the drug liquid is doubtable; drugs for preparation are clinical ones whose excipients may interfere with the final result of judgment; antineoplastic drug sensitivity testing is not applicable to clinical oral drugs; and individual operator error is large.

SUMMARY OF THE PRESENT INVENTION

In order to overcome conventional defects in drug sensitivity test of antineoplastic drugs, the present invention provides a method of improving operation efficiency, flexibility, accuracy and economy of drug sensitivity test of antineoplastic drugs. The technical solution of the present invention is as follows. The present invention is defined in its broadest sense by the appended claims. The present invention provides a method for testing drug sensitivity of antineoplastic drugs, comprising steps of: dissolving an antineoplastic drug in a cell culture fluid comprising tumor cells, in such a manner that the antineoplastic drug fully contacts with the tumor cells; wherein the antineoplastic drug is prepared into a sterile and instantly dissolvable drug membrane for being dissolved in the cell culture fluid as defined in present claim 1. The cell culture fluid is not particularly limited, and may be a conventional cell culture fluid such as RPMI1640, DMEM, 199, MEM, F12, and L-15.

Accordingly, the sterile and instantly dissolvable drug membrane prepared with the antineoplastic drug is easy to dissolve in the cell culture fluid. A thickness and an area thereof are reasonably controlled, for ensuring dissolving speed and mechanical strength, wherein the thickness of the sterile and instantly dissolvable drug membrane is 0.01mm-1mm, preferably 0.04-0.15mm; the area thereof is $0.2cm^2$-$25cm^2$, preferably $0.5cm^2$-$16cm^2$; wherein $1cm^2$ of the sterile and instantly dissolvable drug membrane is dissolved in 1ml of cell culture fluid within 5min, preferably 3min, more preferably 100sec.

Accordingly, the sterile and instantly dissolvable drug membrane comprises the antineoplastic drug with an amount of 0.0001wt%-30wt%, a membrane forming material with an amount of 50wt%-98wt%, and a plasticizer with an amount of 1wt%-20wt%;

wherein the membrane forming material is polyvinyl alcohol, hydroxypropyl methyl cellulose, hydroxyethyl propyl cellulose, sodium carboxymethyl cellulose, or polyvinylpyrrolidone; preferably polyvinyl alcohol, especially polyvinyl alcohol 04-88 or polyvinyl alcohol 05-88; wherein the membrane forming material is water-dissolvable material; in view of manufacturing difficulty and forming performance, the membrane forming material is preferably polyvinyl alcohol, hydroxypropyl methyl cellulose, and polyvinylpyrrolidone; in view of dissolubility, mechanical performance, etc., the membrane forming material is preferably polyvinyl alcohol, especially polyvinyl alcohol 04-88 and polyvinyl alcohol 05-88.

With increase of polymerization, water-dissolubility of the polyvinyl alcohol decreases, and strength thereof after membrane forming increases. Water-dissolubility of polyvinyl alcohol 04-88 and polyvinyl alcohol 05-88 is relatively sufficient, and strength thereof after membrane forming is enough. For drugs which have low contents in the membrane and hardly affects membrane strength, the polyvinyl alcohol 04-88 is sufficient. For drugs which have high contents in the membrane and greatly affects membrane strength, the polyvinyl alcohol 05-88 is used to partly or entirely replace the polyvinyl alcohol 04-88, so as to improve membrane strength.

The plasticizer is glycerol, polyethylene glycol, ethylene glycol, propylene glycol, sorbitol, or triethyl citrate; preferably glycerol.

Accordingly, the antineoplastic drug is not particularly limited, which may be any of the conventional antineoplastic drugs. For example, the antineoplastic drug is selected from a group consisting of fluorouracil, tegafur, tegadifur, doxifluridine, carmofur, methotrexate, busulfan, doxorubicin, daunorubicin, idarubicin, epirubicin, pirarubicin, mitoxantrone, actinomycin D, bleomycin, pingyangmycin, mitomycin, mithramycin, olivomycin, streptozocin, mechlorethamine, chlorambucil, melphalan, cyclophosphamide, ifosfamide, thiotepa, carmustine, lomustine, cytarabine, floxuridine, cyclocylidine, chlorine cyclocylidine, fludarabine, gemcitabine, docetaxel, vinorelbine, vindesine, vincristine, paclitaxel, camptothecin, hydroxy camptothecin, cisplatin, oxaliplatin, carboplatin, nedaplatin, lobaplatin, platinum heptyl, rituximab, ibritumomab, cetuximab, bevacizumab, interleukin-2, leuprorelin acetate, goserelin acetate, anastrozole, letrozole, aminoglutethimide, formestane, exemestane, teniposide, etoposide, pentostatin, irinotecan, sorafenib, capecitabine, decitabine, pemetrexed disodium, procarbazine, harringtonine, thalidomide, dacarbazine, tamoxifen, temozolomide, topotecan, gefitinib, erlotinib, flutamide, nilutamide, bicalutamide, exemestane, arsenite and indirubin.

The cell culture fluid of testing is a liquid culture medium for culturing tumor cells, which comprises amino acid, glucose, inorganic salts, vitamins and trace elements. For stabilizing a pH value, there is generally a pH buff system in the culture fluid, while a small amount of phenol red is added as a pH indicator. Additionally, a certain amount of serum or growth-promoting factors are generally added into the culture fluid. A suitable tumor cell culture fluid may be RPMI1640 (with 10%-20% fetal bovine serum and/or 0.03% glutamine), DMEM, 199, MEM, F12, and L-15; most usually RPMI 1640 with 10%-20% fetal bovine serum. The tumor cells are different from other cells. Therefore, growth-promoting factors are needed during culturing, which is illustrated in Table 1.

Table 1: common growth-promoting factors and final concentration thereof

| Additive | Final concentration |
|---|---|
| BSA | $10^{-5}$mol/ml |
| Transferrin | 5-10$\mu$g/ml |
| Insulin | 5$\mu$g/ml |
| Hydrocortisone | $10^{-6}$mol/ml |
| EGF | 5ng/ml |
| FGF | 5ng/ml |
| NGF | 5ng/ml |

For example, HITES, which is suitable for human small cell lung cancer, is a modified RPMI 1640 culture medium, comprising hydrocortisone, insulin, transferring, estrogen, and selenium.

The tumor cells may be separated tumor tissue obtained by clinical surgical resection, or non-solid tumor cells in vitro separated from blood or body fluid. The tumor cells should be fresh, sterile, timely, and accurate. The cancer tissue should be cultured as soon as possible after be obtained. Usually, within 4 hours, cell survival rate is best. Specimen is stored at 4°C, with no more than 24 hours. It should be noted that during separating, large tumor tissue have degeneration or necrosis areas, which should be avoid; and active areas should be selected. At the same time, because solid tumor tissue may be contaminated during separating, attention should be paid to cleaning and sterilization. For fresh tumor tissue, separating with purely mechanical methods such as pipetting and filtration hardly harms cancer cells, which is suitable for some tumors such as human ovarian cancer and glioma. Cancer tissues are usually solid, wherein tumor cells are embedded in a large number of fiber base materials, and are difficult to be mechanically separated. Therefore, enzyme digestion method is often used to disperse cancer cells. Cancer cells prepared after digestion should have sufficient cell density during culturing. Usually, seeded cell concentration is usually $5 \times 10^5$/ml or $1 \times 10^6$/ml, and culturing time is 37°C.

Another object of the present invention is to provide a use of a sterile and instantly dissolvable drug membrane for testing drug sensitivity of antineoplastic drugs, comprising: an antineoplastic drug with an amount of 0.0001wt%-30wt%, a membrane forming material with an amount of 50wt%-98wt%, and a plasticizer with an amount of 1wt%-20wt%; wherein the membrane forming material is polyvinyl alcohol, hydroxypropyl methyl cellulose, hydroxyethyl propyl cellulose, sodium carboxymethyl cellulose, or polyvinylpyrrolidone; wherein the membrane forming material is water-dissolvable material; in view of manufacturing difficulty and forming performance, the membrane forming material is preferably polyvinyl alcohol, hydroxypropyl methyl cellulose, and polyvinylpyrrolidone, as further defined in present claim 4.

In view of dissolubility, mechanical performance, etc., the membrane forming material is preferably polyvinyl alcohol, especially polyvinyl alcohol 04-88 and polyvinyl alcohol 05-88.

The plasticizer is glycerol, polyethylene glycol, ethylene glycol, propylene glycol, sorbitol, or triethyl citrate; preferably glycerol.

Preferably, the sterile and instantly dissolvable drug membrane comprises an antineoplastic drug with an amount of 0.0001wt%-10wt%, a membrane forming material with an amount of 80wt%-98wt%, and a plasticizer with an amount of 1wt%-10wt%.

The present invention also provides a method for preparing a sterile and instantly dissolvable drug membrane as recited in claims 4 or 5, comprising steps of:

> (1) adding a membrane forming material as defined in claim 4 into water, and stirring or heating for dissolving;
> (2) adding a plasticizer and an antineoplastic drug as defined in claim 5, stirring for thoroughly dissolving, and degassing with heating, staying, or ultrasound;
> (3) coating the solution obtained above on a supporting surface, and drying with hot wind or cold wind; wherein a thickness of the sterile and instantly dissolvable drug membrane is 0.01 mm -1 mm, an area thereof is 0.2 cm2 - 25 cm2 and wherein 1 cm2 of the sterile and instantly dissolvable drug membrane is dissolved in 1 ml of cell culture fluid within 5 min, preferably within 3 min, more preferably within 100 sec;
> (4) stripping and measuring contents thereof, dividing according to a measuring result for obtaining a pre-determined dosage; and
> (5) after dividing, sterilizing with irradiation sterilization or epoxyethane sterilization, preferably the irradiation sterilization.

The sterile and instantly dissolvable drug membrane as above is suitable for testing drug sensitivity of antineoplastic drugs, wherein the sterile and instantly dissolvable drug membrane is directed used in a tumor cell drug sensitivity test, or the sterile and instantly dissolvable drug membrane is dissolved and then activated by adding drug metabolic enzyme, for being used in the tumor cell drug sensitivity test.

Drug sensitivity test of antineoplastic drugs requires that the antineoplastic drug is dissolved in the cell culture fluid, in such a manner that the drug sufficiently contacts with the cells. To ensure that operation is reliable and convenient, the drug must be able to quickly disperse and dissolve in the cell culture fluid. The present invention prepares the sterile and instantly dissolvable drug membrane with the antineoplastic drug for testing drug sensitivity thereof, which is easy to operate and saves costs. Accordingly, the antineoplastic drug is prepared into a drug membrane with exact dosage, good stability, and rapid dissolubility in tumor cell culture fluid. Furthermore, the drug membrane is accurately sliced according to dosages, which is convenient for drug sensitivity test of the antineoplastic drugs.

Dissolution speed test

Providing 10 blank drug membranes with an area of 1cm$^2$ for each group, respectively adding to a 24-well plate, adding 1ml cold RPMI 1640 cell culture fluid (comprising 20% FBS) to each well which has one of the blank drug membranes, softly shaking until the blank drug membranes are fully dissolved; wherein time is recorded and averaged, see Table 2.

Table 2: dissolution speed test (membrane forming material 98%, plasticizer glycerol 2%)

| Membrane thickness (mm) | Dissolving time (s) | | | |
|---|---|---|---|---|
| | polyvinyl alcohol 04-88 | polyvinyl alcohol 05-88 | gelatin | polyvinylpyrrolidone |
| 0.01mm | 23 | 28 | 35 | 18 |
| 0.05mm | 40 | 46 | 58 | 29 |
| 0.10mm | 49 | 61 | 90 | 41 |
| 0.15mm | 56 | 77 | 214 | 53 |
| 0.20mm | 70 | 98 | 322 | 73 |

Stretching performance test

Providing 10 blank drug membranes for each group, providing stretching performance with a universal testing machine, reading and averaging stretching strength and breaking extending rate, which refer to Table 3 and Table 4.

Table 3: stretching strength test of blank drug membranes (membrane forming material 98%, plasticizer glycerol 2%)

| Membrane thickness (mm) | Stretching strength (Mpa) | | | | |
|---|---|---|---|---|---|
| | polyvinyl alcohol 04-88 | polyvinyl alcohol 05-88 | hydroxypropyl methyl cellulose | methacrylic acid copolymer | amioca |
| 0.01mm | 2.1 | 3.7 | 3.2 | 1.5 | 1.2 |
| 0.05mm | 12.8 | 13.5 | 15.6 | 1.9 | 1.7 |
| 0.10mm | 16.9 | 18.8 | 22.9 | 2.8 | 2.3 |
| 0.15mm | 28.5 | 34.2 | 43.1 | 3.1 | 2.9 |
| 0.20mm | 45.7 | 55.2 | 61.4 | 3.7 | 3.2 |

Table. 4: breaking strength test of blank drug membranes (membrane forming material 98%, plasticizer glycerol 2%)

| Membrane thickness (mm) | Breaking extending rate (%) | | | | |
|---|---|---|---|---|---|
| | polyvinyl alcohol 04-88 | polyvinyl alcohol 05-88 | carboxymethylcellulose sodium | propylene oxide | polyacrylic acid |
| 0.01mm | 0.7 | 0.9 | 0.7 | 0.2 | 0.3 |
| 0.05mm | 3.0 | 3.8 | 3.2 | 0.9 | 1.2 |
| 0.10mm | 4.8 | 6.1 | 6.5 | 1.3 | 1.8 |
| 0.15mm | 5.2 | 6.7 | 7.1 | 1.6 | 2.7 |
| 0.20mm | 5.6 | 7.0 | 8.5 | 2.5 | 3.8 |

Table 5: performance test of polyvinyl alcohol blank drug membranes (with a thickness of 0.10mm)

| No. | polyvinyl alcohol (%) | polyvinyl alcohol (%) | glycerol (%) | dissolving time (s) | stretching strength (Mpa) | breaking extending rate (%) |
|---|---|---|---|---|---|---|
| 1 | 99 | -- | 1 | 55 | 19.1 | 2.3 |
| 2 | 96 | -- | 4 | 46 | 14.8 | 6.2 |
| 3 | 90 | -- | 10 | 38 | 10.2 | 7.9 |
| 4 | 80 | -- | 20 | 29 | 6.7 | 8.1 |
| 5 | -- | 99 | 1 | 66 | 22.8 | 3.5 |
| 6 | -- | 96 | 4 | 56 | 16.3 | 6.8 |
| 7 | -- | 90 | 10 | 47 | 12.7 | 8.1 |
| 8 | -- | 80 | 20 | 40 | 9.5 | 8.5 |
| 9 | 70 | 28 | 2 | 48 | 18.5 | 5.6 |
| 10 | 49 | 49 | 2 | 51 | 19.9 | 5.1 |
| 11 | 28 | 70 | 2 | 60 | 19.2 | 6.2 |

DETAILED DESCRIPTION OF THE PREFERRED EMBODIMENT

Preferred embodiment 1:

| | |
|---|---|
| vincristine | 0.04g |
| polyvinyl alcohol 04-88 | 97.96g |
| glycerol | 2g |
| pure water | 200ml |

Heating water to 80°C, adding polyvinyl alcohol 04-88 and stirring for dissolving; cooling to 25°C, adding glycerol and vincristine, stirring for 0.5h; stopping stirring, staying for 4h, and removing air bubbles; coating and drying at 30°C; stripping, cutting and packaging; then sterilizing.

Preferred embodiment 2:

| | |
|---|---|
| etoposide | 1.25g |
| polyvinyl alcohol 05-88 | 96.75g |
| glycerol | 2g |
| pure water | 400ml |

Heating water to 80°C, adding polyvinyl alcohol 05-88 and stirring for dissolving; cooling to 25°C, adding glycerol and etoposide, stirring for 0.5h; stopping stirring, staying for 4h, and removing air bubbles; coating and drying at 30°C; stripping, cutting and packaging; then sterilizing.

Preferred embodiment 3:

| | |
|---|---|
| methotrexate | 0.4g |
| polyvinyl alcohol 04-88 | 97.6g |
| polyethylene glycol | 2g |

(continued)

| | |
|---|---|
| pure water | 200ml |

Heating water to 80°C, adding polyvinyl alcohol 04-88 and stirring for dissolving; cooling to 25°C, adding polyethylene glycol and methotrexate, stirring for 0.5h; stopping stirring, staying for 4h, and removing air bubbles; coating and drying at 30°C; stripping, cutting and packaging; then sterilizing.

Preferred embodiment 4:

| | |
|---|---|
| cytarabine | 0.5g |
| hydroxypropyl methyl cellulose | 97.5g |
| glycerol | 2g |
| pure water | 200ml |

Heating water to 80°C, adding hydroxypropyl methyl cellulose and stirring for dissolving; cooling to 25°C, adding glycerol and cytarabine, stirring for 0.5h; stopping stirring, staying for 4h, and removing air bubbles; coating and drying at 30°C; stripping, cutting and packaging; then sterilizing.

Preferred embodiment 5:

| | |
|---|---|
| hydroxy camptothecin | 0.01g |
| sodium carboxymethyl cellulose | 97.99g |
| glycerol | 2g |
| pure water | 400ml |

Heating water to 80°C, adding sodium carboxymethyl cellulose and stirring for dissolving; cooling to 70°C, adding glycerol and hydroxy camptothecin, stirring for 0.5h; stopping stirring, keeping temperature for 1h, and removing air bubbles; coating and drying at 70°C; stripping, cutting and packaging; then sterilizing.

Preferred embodiment 6:

| | |
|---|---|
| homoharringtonine | 0.0012g |
| polyvinyl alcohol 05-88 | 97.999g |
| ethylene glycol | 2g |
| pure water | 400ml |

Heating water to 80°C, adding polyvinyl alcohol 05-88 and stirring for dissolving; cooling to 25°C, adding ethylene glycol and homoharringtonine, stirring for 0.5h; stopping stirring, staying for 4h, and removing air bubbles; coating and drying at 30°C; stripping, cutting and packaging; then sterilizing.

Preferred embodiment 7:

| | |
|---|---|
| daunorubicin | 0.008g |
| polyvinylpyrrolidone | 97.992g |
| glycerol | 2g |
| pure water | 200ml |

Heating water to 80°C, adding polyvinylpyrrolidone and stirring for dissolving; cooling to 25°C, adding glycerol and daunorubicin, stirring for 0.5h; stopping stirring, staying for 4h, and removing air bubbles; coating and drying at 30°C; stripping, cutting and packaging; then sterilizing.

Preferred embodiment 8:

| | |
|---|---|
| mitoxantrone | 0.005g |
| polyvinyl alcohol 04-88 | 97.995g |
| triethyl citrate | 2g |
| pure water | 400ml |

Heating water to 80°C, adding polyvinyl alcohol 04-88 and stirring for dissolving; cooling to 25°C, adding triethyl citrate and mitoxantrone, stirring for 0.5h; stopping stirring, staying for 4h, and removing air bubbles; coating and drying at 30°C; stripping, cutting and packaging; then sterilizing.

Preferred embodiment 9:

| | |
|---|---|
| doxorubicin | 0.2g |
| polyvinyl alcohol 05-88 | 97.8g |
| propylene glycol | 2g |
| pure water | 200ml |

Heating water to 80°C, adding polyvinyl alcohol 05-88 and stirring for dissolving; cooling to 25°C, adding propylene glycol and doxorubicin, stirring for 0.5h; stopping stirring, staying for 4h, and removing air bubbles; coating and drying at 30°C; stripping, cutting and packaging; then sterilizing.

Preferred embodiment 10:

| | |
|---|---|
| idarubicin | 0.05g |
| polyvinyl alcohol 04-88 | 97.95g |
| glycerol | 2g |
| pure water | 200ml |

Heating water to 80°C, adding polyvinyl alcohol 04-88 and stirring for dissolving; cooling to 25°C, adding glycerol and idarubicin, stirring for 0.5h; stopping stirring, staying for 4h, and removing air bubbles; coating and drying at 30°C; stripping, cutting and packaging; then sterilizing.

Preferred embodiment 11:

| | |
|---|---|
| polyvinyl alcohol 04-88 | 98g |
| glycerol | 2g |
| pure water | 200ml |

Heating water to 80°C, adding polyvinyl alcohol 04-88 and stirring for dissolving; cooling to 70°C, adding glycerol, stirring for 0.5h; stopping stirring, staying for 1h, and removing air bubbles; coating and drying at 70°C; stripping, cutting and packaging; then sterilizing.

Preferred embodiment 12:

Drug sensitivity of antineoplastic drugs

Selecting chemotherapy drugs according to treatment guidelines of acute myeloid leukemia, preparing drug membranes according to natures of the drugs and peak plasma concentration of the drugs in human body, and preparing blank drug membranes according to preferred embodiments 1-11; according to measuring results, cutting the drug membrane for comprising the chemotherapy drug with 1ml peak plasma concentration, and cutting blank drug membrane into $1cm^2$.

Respectively acquiring 10ml marrow (heparin) from 20 patients with acute myeloid leukemia with aseptic techniques before chemotherapy, separating leukemia cells with Ficoll in a cleaned room, washing with culture fluid and putting in RPMI 1640 culture system for obtaining $1 \times 10^6$/ml cell suspension.

Seeding to cell suspension in a 24-well plate (with lml/well), providing a cell control group (tumor cell suspension), a blank drug membrane control group (blank drug membrane + tumor cell suspension), and an experimental group (tumor cell suspension + chemotherapy drug membrane); wherein each drugs in the experimental groups are added in three wells, and no chemotherapy drug is added to the wells for the control group; the experimental group comprises a single-drug group and a combined-drug group (referring to Table 6); when using combined-drugs, combining corresponding single-drugs according to a chemotherapy regimen, and placing in a same well; adding corresponding drug membrane to each well as pre-determined, and adding the cell suspension with lml/well, gently shaking until the blank drug membranes are fully dissolved; culturing at 37°C with 5% carbon dioxide and saturated humidity for 24 hours; collecting suspension cells with centrifugation, wherein a micro-centrifuge speed is 2000RPM, and a centrifugation time is 5min; the culture medium is discarded; washing the cells twice with cold PBS (2000RPM, centrifugation time 5min for collecting cells); buffing the suspension cells with 400μl IX Binding Buffer, wherein a concentration is about $1 \times 10^6$/ml; adding 5μl Annexin V-FITC in the cell suspension and gently mixing, then incubating at 2-8°C in dark for 15min; adding 10μl PI and gently mixing, then incubating at 2-8°C in dark for 5min; measuring apoptosis and necrosis of the drug control group and the experimental group within one hour by a flow cytometry, so as to calculate inhibition rate of drugs and drug sensitivity.

Table 6: drug sensitivity according to test

| Drug | Sensitivity (%) |
|---|---|
| vincristine membrane | 25 |
| etoposide membrane | 10 |
| methotrexate membrane | 0 |
| cytarabine membrane | 35 |
| hydroxy camptothecin membrane | 25 |
| homoharringtonine membrane | 35 |
| daunorubicin membrane | 65 |
| mitoxantrone membrane | 25 |
| doxorubicin membrane | 40 |
| idarubicin membrane | 30 |
| cytarabine membrane + daunorubicin membrane | 95 |
| cytarabine membrane + idarubicin membrane | 70 |
| cytarabine membrane + doxorubicin membrane | 85 |
| cytarabine membrane + homoharringtonine membrane | 85 |

(inhibition rate = [(active cells of blank drug membrane control group – active cells of experimental group) / active cells of blank drug membrane control group] × 100%; wherein inhibition rate > 50% means sensitive)

Preferred embodiment 13:

Selecting chemotherapy drugs according to treatment guidelines of small cell lung cancer, preparing drug membranes according to natures of the drugs and peak plasma concentration of the drugs in human body, and preparing blank drug membranes according to preferred embodiments 1-11; according to measuring results, cutting the drug membrane for comprising the chemotherapy drug with 1ml peak plasma concentration, and cutting blank drug membrane into $1cm^2$.

Before chemotherapy, taking a fresh small cell lung cancer tissue block ($0.5cm^3$-$1cm^3$) from a patient, placing in sterile saline (containing 200,000 units/ml penicillin, 250,000 units/ml streptomycin), cutting off fat, fiber and other normal tissue on an ultra clean table, as well as necrosis and bloody tissues; washing with saline comprising antibiotics, adding tissue digestive enzymes (such as trypsin and collagenase) for digesting for 2-3h, in such a manner that tissue blocks becomes individual cells and pass through a copper mesh; centrifuging for removing supernatant, adding RPMI 1640 culture medium for pipetting into cell suspension, counting and adjusting cell concentration to $1\times10^6$/ml.

Seeding to cell suspension in a 24-well plate (with lml/well), providing a cell control group (tumor cell suspension), a blank drug membrane control group (blank drug membrane + tumor cell suspension), and an experimental group (tumor cell suspension + chemotherapy drug membrane); wherein each drugs in the experimental groups are added in three wells, and no chemotherapy drug is added to the wells for the control group; the experimental group comprises a single-drug group and a combined-drug group; when using combined-drugs, combining corresponding single-drugs according to a chemotherapy regimen, and placing in a same well; adding corresponding drug membrane and activating enzyme to each well as pre-determined, and adding the cell suspension with lml/well, gently shaking until the blank drug membranes are fully dissolved; culturing at 37°C with 5% carbon dioxide and saturated humidity for 24 hours; collecting suspension cells with centrifugation, wherein a micro-centrifuge speed is 2000RPM, and a centrifugation time is 5min; the culture medium is discarded; washing the cells twice with cold PBS (2000RPM, centrifugation time 5min for collecting cells); buffing the suspension cells with 400μl IX Binding Buffer, wherein a concentration is about $1\times10^6$/ml; adding 5μl Annexin V-FITC in the cell suspension and gently mixing, then incubating at 2-8°C in dark for 15min; adding 10μl PI and gently mixing, then incubating at 2-8°C in dark for 5min; measuring apoptosis and necrosis of the drug control group and the experimental group within one hour by a flow cytometry, so as to calculate inhibition rate of drugs.

Table 7: chemotherapy regimens and inhibition rates

| (when using combined-drugs, combining corresponding single-drugs according to a chemotherapy regimen, and adding corresponding corresponding activating enzyme) | |
| --- | --- |
| Regimen | Inhibition rate (%) |
| etoposide membrane | 76 |
| paclitaxel membrane | 69 |
| topotecan membrane | 61 |
| etoposide membrane + cisplatin membrane | 90 |
| etoposide membrane + carboplatin membrane | 83 |
| irinotecan membrane + cisplatin membrane | 80 |
| carboplatin membrane + paclitaxel membrane + etoposide membrane | 96 |
| cyclophosphamide membrane + doxorubicin membrane + vincristine membrane + hepatomicrosome enzyme | 85 |
| cyclophosphamide membrane + doxorubicin membrane + etoposide membrane + hepatomicrosome enzyme | 84 |
| membrane + hepatomicrosome enzyme | |

(inhibition rate = [(active cells of blank drug membrane control group – active cells of experimental group) / active cells of blank drug membrane control group] × 100%; wherein cyclophosphamide should be activated by hepatomicrosome enzyme)

Preferred embodiment 14:

Selecting chemotherapy drugs according to treatment guidelines of mammary cancer, preparing drug membranes according to natures of the drugs and peak plasma concentration of the drugs in human body, and preparing blank drug membranes according to preferred embodiments 1-11; according to measuring results, cutting the drug membrane for comprising the chemotherapy drug with 1ml peak plasma concentration, and cutting blank drug membrane into $1cm^2$.

Immersing a surgically removed mammary cancer tissue in sterile Hanks liquid, adding a few normal tissues into a RPMI 1640 culture medium comprising 1% double-antibody and washing repeatedly, cutting into $1\text{-}2mm^3$ blocks; adding 10 times 0.25% trypsin solution and 0.02% elhylene diamine tetraacetic acid (EDTA), digesting at 37°C for 40min, shanking once every 5min, removing digesting liquid and adding serum-free 1640 culture medium for washing, then adding a little culture fluid and pipetting with a suction tube, in such a manner that single cells are formed and pass though a 40 mesh net; and preparing into $1\times10^6$/ml suspension with RPMI 1640 culture system.

Seeding to cell suspension in a 24-well plate (with lml/well), providing a cell control group (tumor cell suspension), a blank drug membrane control group (blank drug membrane + tumor cell suspension), and an experimental group (tumor cell suspension + chemotherapy drug membrane); wherein each drugs in the experimental groups are added in three wells, and no chemotherapy drug is added to the wells for the control group; when using combined-drugs, combining corresponding single-drugs according to a chemotherapy regimen, and placing in a same well; adding corresponding drug membrane and activating enzyme to each well as pre-determined, and adding the cell suspension with 1 ml/well, gently shaking until the blank drug membranes are fully dissolved; placing the 24-well plate in a 5% $CO_2$ incubator at 37°C, adding 20$\mu$l MTT to each well and gently mixing, incubating for 6-12h, taking the 24-well plate and centrifuging for 15min with a speed of 1000r/min, adding 100$\mu$l DMSO into each well, thoroughly shaking until purple crystal in each well is fully dissolved; then analyzing absorbance (A) value of each well at 570nm with an enzyme labeled analyzer.

Inhibition rates of different chemotherapy drugs on mammary cancer cells are calculated as follows:

$$\text{mammary cancer cell inhibition rate} = (1 - \text{A value of experimental group} / \text{A value of blank drug membrane control group}) \times 100\%.$$

Table 8: chemotherapy regimens and inhibition rates

| (when using combined-drugs, combining corresponding single-drugs according to a chemotherapy regimen, and adding corresponding corresponding activating enzyme) | |
| --- | --- |
| Regimen | Inhibition rate (%) |
| cyclophosphamide membrane + doxorubicin membrane + docetaxel membrane + hepatomicrosome enzyme | 83 |
| cyclophosphamide membrane + doxorubicin membrane + paclitaxel membrane + hepatomicrosome enzyme | 76 |
| cyclophosphamide membrane + docetaxel membrane + hepatomicrosome enzyme | 78 |
| cyclophosphamide membrane + doxorubicin membrane + hepatomicrosome enzyme | 55 |
| cyclophosphamide membrane + doxorubicin membrane + fluorouracil membrane + hepatomicrosome enzyme | 71 |
| cyclophosphamide membrane + methotrexate membrane + fluorouracil membrane + hepatomicrosome enzyme | 63 |
| cyclophosphamide membrane +epirubicin membrane+ hepatomicrosome enzyme | 79 |

(inhibition rate = [(active cells of blank drug membrane control group – active cells of experimental group) / active cells of blank drug membrane control group] × 100%).

**Claims**

1. A method for testing sensitivity of tumor cells to antineoplastic drugs, comprising the steps of: dissolving an antineoplastic drug in a cell culture fluid comprising the tumor cells, wherein the antineoplastic drug is comprised in a sterile and instantly dissolvable drug membrane, and wherein said membrane is fully dissolved in the cell culture fluid, and wherein the thickness of the sterile and instantly dissolvable drug membrane is 0.01 mm - 1 mm and the area thereof is 0.2 cm$^2$-25 cm$^2$, and wherein 1 cm$^2$ of the sterile and instantly dissolvable drug membrane is dissolved in 1 ml of the cell culture fluid within 5 min, preferably within 3 min and more preferably within 100 sec,

   and assessing the inhibition rate of the drug on the tumor cells, wherein an inhibition rate > 50% means that the tumor cells are sensitive to the drug,

   and wherein said inhibition rate is defined as [(active cells of blank drug membrane control group - active cells of experimental group) / active cells of blank drug membrane control group] × 100%.

2. The method, as recited in claim 1, wherein the sterile and instantly dissolvable drug membrane comprises the antineoplastic drug with an amount of 0.0001wt%-30wt%, a membrane forming material with an amount of 50wt% - 98wt%, and a plasticizer with an amount of 1wt% -20wt%.

3. The method, as recited in claim 1 or 2, wherein the antineoplastic drug is selected from a group consisting of fluorouracil, tegafur, tegadifur, doxifluridine, carmofur, methotrexate, busulfan, doxorubicin, daunorubicin, idarubicin, epirubicin, pirarubicin, mitoxantrone, actinomycin D, bleomycin, pingyangmycin, mitomycin, mithramycin, olivomycin, streptozocin, mechlorethamine, chlorambucil, melphalan, cyclophosphamide, ifosfamide, thiotepa, carmustine, lomustine, cytarabine, floxuridine, cyclocylidine, chlorine cyclocylidine, fludarabine, gemcitabine, docetaxel, vinorelbine, vindesine, vincristine, paclitaxel, camptothecin, hydroxy camptothecin, cisplatin, oxaliplatin, carboplatin, nedaplatin, lobaplatin, platinum heptyl, rituximab, ibritumomab, cetuximab, bevacizumab, interleukin-2, leuprorelin acetate, goserelin acetate, anastrozole, letrozole, aminoglutethimide, formestane, exemestane, teniposide, etoposide, pentostatin, irinotecan, sorafenib, capecitabine, decitabine, pemetrexed disodium, procarbazine, harringtonine, thalidomide, dacarbazine, tamoxifen, temozolomide, topotecan, gefitinib, erlotinib, flutamide, nilutamide, bicalutamide, exemestane, arsenite and indirubin.

4. Use of a sterile and instantly dissolvable drug membrane for testing drug sensitivity of antineoplastic drugs, said membrane comprising: an antineoplastic drug with an amount of 0.0001wt% - 30wt%, a membrane forming material with an amount of 50wt% - 98wt%, and a plasticizer with an amount of 1wt% - 20wt%; wherein the membrane forming material is polyvinyl alcohol, hydroxypropyl methyl cellulose, hydroxyethyl propyl cellulose, sodium carboxymethyl cellulose, or polyvinylpyrrolidone; the plasticizer is glycerol, polyethylene glycol, ethylene glycol, propylene glycol, sorbitol, or triethyl citrate and wherein a thickness of the sterile and instantly dissolvable drug membrane is 0.01 mm - 1 mm, the area thereof is 0.2 cm$^2$ - 25cm$^2$; wherein 1 cm$^2$ of the sterile and instantly dissolvable drug membrane is dissolved in 1 ml of cell culture fluid within 5 min, preferably within 3 min and more preferably within 100 sec.

5. The use, as recited in claim 4, wherein the antineoplastic drug is selected from a group consisting of fluorouracil, tegafur, tegadifur, doxifluridine, carmofur, methotrexate, busulfan, doxorubicin, daunorubicin, idarubicin, epirubicin, pirarubicin, mitoxantrone, actinomycin D, bleomycin, pingyangmycin, mitomycin, mithramycin, olivomycin, streptozocin, mechlorethamine, chlorambucil, melphalan, cyclophosphamide, ifosfamide, thiotepa, carmustine, lomustine, cytarabine, floxuridine, cyclocylidine, chlorine cyclocylidine, fludarabine, gemcitabine, docetaxel, vinorelbine, vindesine, vincristine, paclitaxel, camptothecin, hydroxy camptothecin, cisplatin, oxaliplatin, carboplatin, nedaplatin, lobaplatin, platinum heptyl, rituximab, ibritumomab, cetuximab, bevacizumab, interleukin-2, leuprorelin acetate, goserelin acetate, anastrozole, letrozole, aminoglutethimide, formestane, exemestane, teniposide, etoposide, pentostatin, irinotecan, sorafenib, capecitabine, decitabine, pemetrexed disodium, procarbazine, harringtonine, thalidomide, dacarbazine, tamoxifen, temozolomide, topotecan, gefitinib, erlotinib, flutamide, nilutamide, bicalutamide, exemestane, arsenite and indirubin.

6. A method for preparing a sterile and instantly dissolvable drug membrane as recited in claim 4 or 5, comprising steps of:

   adding a membrane forming material as defined in claim 4 into water, and stirring or heating for dissolving;
   adding a plasticizer and an antineoplastic drug as defined in claim 5, stirring for thoroughly dissolving, and degassing with heating, staying or ultrasound;
   coating the solution obtained above on a supporting surface, and drying with hot wind or cold wind; wherein a thickness of the sterile and instantly dissolvable drug membrane is 0.01 mm -1 mm, an area thereof is 0.2 cm$^2$ - 25 cm$^2$ and wherein 1 cm$^2$ of the sterile and instantly dissolvable drug membrane is dissolved in 1 ml of cell culture fluid within 5 min, preferably within 3 min, more preferably within 100 sec. stripping and measuring contents thereof, dividing according to a measuring result for obtaining a pre-determined dosage; and

after dividing, sterilizing with irradiation sterilization or epoxyethane sterilization, preferably the irradiation sterilization.

7. The method, as recited in claim 6, wherein the sterile and instantly dissolvable drug membrane is directly used in a tumor cell drug sensitivity test, or the sterile and instantly dissolvable drug membrane is dissolved and then activated by adding drug metabolic enzyme, for being used in the tumor cell drug sensitivity test.


**Patentansprüche**

1. Verfahren zum Testen der Empfindlichkeit von Tumorzellen auf antineoplastische Wirkstoffe, umfassend die folgenden Schritte:

Lösen eines antineoplastischen Wirkstoffs in einem Zellkulturfluid, das die Tumorzellen umfasst, wobei der antineoplastische Wirkstoff in einer sterilen und sofort löslichen Wirkstoffmembran enthalten ist, und wobei die Membran im Zellkulturfluid vollständig gelöst wird, und wobei die Dicke der sterilen und sofort löslichen Wirkstoffmembran 0,01 mm - 1 mm beträgt und die Fläche davon 0,2 cm$^2$ - 25 cm$^2$ beträgt, und wobei 1 cm$^2$ der sterilen und sofort löslichen Wirkstoffmembran in 1 ml des Zellkulturfluids innerhalb von 5 min, vorzugsweise innerhalb von 3 min und bevorzugter innerhalb von 100 s gelöst wird,

und Bewerten der Inhibierungsrate des Wirkstoffs auf die Tumorzellen, wobei eine Inhibierungsrate > 50 % bedeutet, dass die Tumorzellen auf den Wirkstoff empfindlich sind,

und wobei die Inhibierungsrate definiert ist als [(aktive Zellen einer Kontrollgruppe mit leerer Wirkstoffmembran - aktive Zellen der Experimentalgruppe) / aktive Zellen der Kontrollgruppe mit leerer Wirkstoffmembran] $\times$ 100 %.

2. Verfahren nach Anspruch 1, wobei die sterile und sofort lösliche Wirkstoffmembran den antineoplastischen Wirkstoff in einer Menge von 0,0001 Gew.-% - 30 Gew.-%, ein membranbildendes Material in einer Menge von 50 Gew.-% - 98 Gew.-% und einen Weichmacher in einer Menge von 1 Gew.-% - 20 Gew.-% umfasst.

3. Verfahren nach Anspruch 1 oder 2, wobei der antineoplastische Wirkstoff aus einer Gruppe ausgewählt ist, bestehend aus Fluoruracil, Tegafur, Tegadifur, Doxifluridin, Carmofur, Methotrexat, Busulfan, Doxorubicin, Daunorubicin, Idarubicin, Epirubicin, Pirarubicin, Mitoxantron, Actinomycin D, Bleomycin, Pingyangmycin, Mitomycin, Mithramycin, Olivomycin, Streptozocin, Mechlorethamin, Chlorambucil, Melphalan, Cyclophosphamid, Ifosfamid, Thiotepa, Carmustin, Lomustin, Cytarabin, Floxuridin, Cyclocylidin, Chlorcyclocylidin, Fludarabin, Gemcitabin, Docetaxel, Vinorelbin, Vindesin, Vincristin, Paclitaxel, Camptothecin, Hydroxycamptothecin, Cisplatin, Oxaliplatin, Carboplatin, Nedaplatin, Lobaplatin, Platinheptyl, Rituximab, Ibritumomab, Cetuximab, Bevacizumab, Interleukin-2, Leuprorelinacetat, Goserelinacetat, Anastrozol, Letrozol, Aminoglutethimid, Formestan, Exemestan, Teniposid, Etoposid, Pentostatin, Irinotecan, Sorafenib, Capecitabin, Decitabin, Pemetrexed-Dinatrium, Procarbazin, Harringtonin, Thalidomid, Dacarbazin, Tamoxifen, Temozolomid, Topotecan, Gefitinib, Erlotinib, Flutamid, Nilutamid, Bicalutamid, Exemestan, Arsenit und Indirubin.

4. Verwendung einer sterilen und sofort löslichen Wirkstoffmembran zum Testen der Wirkstoffempfindlichkeit auf antineoplastische Wirkstoffe, wobei die Membran Folgendes umfasst: einen antineoplastischen Wirkstoff in einer Menge von 0,0001 Gew.-% - 30 Gew.-%, ein membranbildendes Material in einer Menge von 50 Gew.-% - 98 Gew.-% und einen Weichmacher in einer Menge von 1 Gew.-% - 20 Gew.-%; wobei das membranbildende Material Polyvinylalkohol, Hydroxypropylmethylcellulose, Hydroxyethylpropylcellulose, Natriumcarboxymethylcellulose oder Polyvinylpyrrolidon ist; der Weichmacher Glycerin, Polyethylenglycol, Ethylenglycol, Propylenglycol, Sorbitol oder Triethylcitrat ist, und wobei eine Dicke der sterilen und sofort löslichen Wirkstoffmembran 0,01 mm - 1 mm beträgt, die Fläche davon 0,2 cm$^2$ - 25 cm$^2$ beträgt; wobei 1 cm$^2$ der sterilen und sofort löslichen Wirkstoffmembran in 1 ml eines Zellkulturfluids innerhalb von 5 min, vorzugsweise innerhalb von 3 min und bevorzugter innerhalb von 100 s gelöst wird.

5. Verwendung nach Anspruch 4, wobei der antineoplastische Wirkstoff aus einer Gruppe ausgewählt ist, bestehend aus Fluoruracil, Tegafur, Tegadifur, Doxifluridin, Carmofur, Methotrexat, Busulfan, Doxorubicin, Daunorubicin, Idarubicin, Epirubicin, Pirarubicin, Mitoxantron, Actinomycin D, Bleomycin, Pingyangmycin, Mitomycin, Mithramycin, Olivomycin, Streptozocin, Mechlorethamin, Chlorambucil, Melphalan, Cyclophosphamid, Ifosfamid, Thiotepa, Carmustin, Lomustin, Cytarabin, Floxuridin, Cyclocylidin, Chlorcyclocylidin, Fludarabin, Gemcitabin, Docetaxel, Vinorelbin, Vindesin, Vincristin, Paclitaxel, Camptothecin, Hydroxycamptothecin, Cisplatin, Oxaliplatin, Carboplatin, Nedaplatin, Lobaplatin, Platinheptyl, Rituximab, Ibritumomab, Cetuximab, Bevacizumab, Interleukin-2, Leuprorelinacetat, Goserelinacetat, Anastrozol, Letrozol, Aminoglutethimid, Formestan, Exemestan, Teniposid, Etoposid, Pentostatin, Irinotecan, Sorafenib, Capecitabin, Decitabin, Pemetrexed-Dinatrium, Procarbazin, Harringtonin, Thalidomid, Dacarbazin, Tamoxifen, Temozolomid, Topotecan, Gefitinib, Erlotinib, Flutamid, Nilutamid, Bicalutamid, Exemestan, Arsenit und Indirubin.

6. Verfahren zum Herstellen einer sterilen und sofort löslichen Wirkstoffmembran nach Anspruch 4 oder 5, umfassend die folgenden Schritte:

Hinzufügen eines membranbildenden Materials nach Anspruch 4 zu Wasser, und Rühren oder Erwärmen zum Lösen;
Hinzufügen eines Weichmachers und eines antineoplastischen Wirkstoffs nach Anspruch 5, Rühren zum vollständigen Lösen und Entgasen durch Erwärmen, Stehenlassen oder Ultraschall;
Beschichten einer Trägerfläche mit der oben erhaltenen Lösung, und Trocknen mit heißem Wind oder kaltem Wind;
wobei eine Dicke der sterilen und sofort löslichen Wirkstoffmembran 0,01 mm - 1 mm beträgt, eine Fläche davon 0,2 cm$^2$ - 25 cm$^2$ beträgt und wobei 1 cm$^2$ der sterilen und sofort löslichen Wirkstoffmembran in 1 ml eines Zellkulturfluids innerhalb von 5 min, vorzugsweise innerhalb von 3 min, bevorzugter innerhalb von 100 s gelöst wird;
Stripping und Messen des Gehalts davon, Aufteilen gemäß einem Messergebnis zum Erhalten einer vorbestimmten Dosierung; und
nach dem Aufteilen, Sterilisieren durch Strahlensterilisation oder Epoxyethansterilisation, vorzugsweise die Strahlensterilisation.

7. Verfahren nach Anspruch 6, wobei die sterile und sofort lösliche Wirkstoffmembran direkt in einem Wirkstoffempfindlichkeitstest von Tumorzellen verwendet wird, oder die sterile und sofort lösliche Wirkstoffmembran gelöst und dann durch das Hinzufügen eines Stoffwechselenzyms des Wirkstoffs aktiviert wird, um in dem Wirkstoffempfindlichkeitstest von Tumorzellen verwendet zu werden.

**Revendications**

1. Procédé de test de la sensibilité de cellules tumorales à des médicaments antinéoplasiques, comprenant les étapes suivantes : la dissolution d'un médicament antinéoplasique dans un fluide de culture cellulaire comprenant les cellules tumorales, dans lequel le médicament antinéoplasique est compris dans une membrane pour médicament stérile et à dissolution instantanée, et dans lequel ladite membrane est dissoute totalement dans le fluide de culture cellulaire, et dans lequel l'épaisseur de la membrane pour médicament stérile et à dissolution instantanée est de 0,01 mm à 1 mm et la surface de celle-ci est de 0,2 cm$^2$ à 25 cm$^2$, et dans lequel 1 cm$^2$ de la membrane pour médicament stérile et à dissolution instantanée est dissoute dans 1 ml du fluide de culture cellulaire en moins de 5 min, de préférence en moins de 3 min et de manière davantage préférée en moins de 100 sec, et l'évaluation du taux d'inhibition du médicament sur les cellules tumorales, dans lequel un taux d'inhibition > 50 % signifie que les cellules tumorales sont sensibles au médicament, et dans lequel ledit taux d'inhibition est défini par [(cellules actives du groupe témoin à membrane pour médicament à blanc - cellules actives du groupe expérimental) / cellules actives du groupe témoin à membrane pour médicament à blanc] x 100 %

2. Procédé, selon la revendication 1, dans lequel la membrane pour médicament stérile et à dissolution instantanée comprenant le médicament antinéoplasique en une quantité de 0,0001 % en poids à 30 % en poids, un matériau formant membrane en une quantité de 50 % en poids à 98 % en poids, et un plastifiant en une quantité de 1 % en poids à 20 % en poids.

3. Procédé, selon la revendication 1 ou 2, dans lequel le médicament antinéoplasique est sélectionné dans un groupe constitué du fluorouracile, du tégafur, du tégadifur, de la doxifluridine, du carmofur, du méthotrexate, du busulfan, de la doxorubicine, de la daunorubicine, de l'idarubicine, de l'épirubicine, de la pirarubicine, de la mitoxantrone, de l'actinomycine D, de la bléomycine, de la pingyangmycine, de la mitomycine, de la mithramycine, de l'olivomycine, de la streptozocine, de la méchloréthamine, du chlorambucil, du melphalan, du cyclophosphamide, de l'ifosfamide, du thiotépa, de la carmustine, de la lomustine, de la cytarabine, de la floxuridine, de la cyclocylidine, de la cyclocylidine chlore, de la fludarabine, de la gemcitabine, du docétaxel, de la vinorelbine, de la vindésine, de la vincristine, du paclitaxel, de la camptothécine, de l'hydroxy camptothécine, du cisplatine, de l'oxaliplatine, du carboplatine, du nédaplatine, du lobaplatine, du platine heptyle, du rituximab, de l'ibritumomab, du cétuximab, du bévacizumab, de l'interleukine-2, de l'acétate de leuproréline, de l'acétate de goséréline, de l'anastrozole, du létrozole, de l'aminoglutéthimide, du formestane, de l'exémestane, du téniposide, de l'étoposide, de la pentostatine, de l'irinotécan, du sorafénib, de la capécitabine, de la décitabine, du pémétrexed disodium, de la procarbazine, de l'harringtonine, du thalidomide, de la dacarbazine, du tamoxifène, du témozolomide, du topotécan, du géfitinib, de l'erlotinib, du flutamide, du nilutamide, du bicalutamide, de l'exémestane, de l'arsenite et de l'indirubine.

4. Utilisation d'une membrane pour médicament stérile et à dissolution instantanée pour tester la sensibilité médicamenteuse de médicaments antinéoplasiques, ladite membrane comprenant : un médicament antinéoplasique en une quantité de 0,0001 % en poids à 30 % en poids, un matériau formant membrane en une quantité de 50 % en poids à 98 % en poids, et un plastifiant en une quantité de 1 % en poids à 20 % en poids ; dans laquelle le matériau formant membrane est un polyalcool vinylique, une hydroxypropyl méthylcellulose, une hydroxyéthyl propyl cellulose, une carboxyméthyl cellulose sodique, ou une polyvinylpyrrolidone ; le plastifiant est le glycérol, un polyéthylène glycol, l'éthylène glycol, le propylène glycol, le sorbitol, ou le citrate de triéthyle et dans laquelle une épaisseur de la membrane pour médicament stérile et à dissolution instantanée est de 0,01 mm à 1 mm, la surface de celle-ci est de 0,2 cm$^2$ à 25 cm$^2$ ; dans laquelle 1 cm$^2$ de la membrane pour médicament stérile et à dissolution instantanée est dissoute dans 1 ml du fluide de culture cellulaire en moins de 5 min, de préférence en moins de 3 min et de manière davantage préférée en moins de 100 sec.

5. Utilisation, selon la revendication 4, dans laquelle le médicament antinéoplasique est sélectionné dans un groupe constitué du fluorouracile, du tégafur, du tégadifur, de la doxifluridine, du carmofur, du méthotrexate, du busulfan, de la doxorubicine, de la daunorubicine, de l'idarubicine, de l'épirubicine, de la pirarubicine, de la mitoxantrone, de l'actinomycine D, de la bléomycine, de la pingyangmycine, de la mitomycine, de la mithramycine, de l'olivomycine, de la streptozocine, de la méchloréthamine, du chlorambucil, du melphalan, du cyclophosphamide, de l'ifosfamide, du thiotépa, de la carmustine, de la lomustine, de la cytarabine, de la floxuridine, de la cyclocylidine, de la cyclocylidine chlore, de la fludarabine, de la gemcitabine, du docétaxel, de la vinorelbine, de la vindésine, de la vincristine, du paclitaxel, de la camptothécine, de l'hydroxy camptothécine, du cisplatine, de l'oxaliplatine, du carboplatine, du nédaplatine, du lobaplatine, du platine heptyle, du rituximab, de l'ibritumomab, du cétuximab, du bévacizumab, de l'interleukine-2, de l'acétate de leuproréline, de l'acétate de goséréline, de l'anastrozole, du létrozole, de l'aminoglutéthimide, du formestane, de l'exémestane, du téniposide, de l'étoposide, de la pentostatine, de l'irinotécan, du sorafénib, de la capécitabine, de la décitabine, du pémétrexed disodium, de la procarbazine, de l'harringtonine, du thalidomide, de la dacarbazine, du tamoxifène, du témozolomide, du topotécan, du géfitinib, de l'erlotinib, du flutamide, du nilutamide, du bicalutamide, de l'exémestane, de l'arsenite et de l'indirubine.

6. Procédé de préparation d'une membrane pour médicament stérile et à dissolution instantanée selon la revendication 4 ou 5, comprenant les étapes suivantes :

l'ajout d'un matériau formant membrane tel que défini dans la revendication 4 à de l'eau, et une agitation ou un chauffage à des fins de dissolution ;
l'ajout d'un plastifiant et d'un médicament antinéoplasique tels que définis dans la revendication 5, une agitation à des fins de dissolution complète, et un dégazage avec chauffage, au repos ou sous ultrasons ;
le revêtement de la solution obtenue sur une surface de support, et un séchage avec un vent chaud ou un vent froid ;
dans lequel une épaisseur de la membrane pour médicament stérile et à dissolution instantanée est de 0,01 mm à 1 mm, une surface de celle-ci est de 0,2 cm$^2$ à 25 cm$^2$ et dans lequel 1 cm$^2$ de la membrane pour médicament stérile et à dissolution instantanée est dissoute dans 1 ml du fluide de culture cellulaire en moins de 5 min, de préférence en moins de 3 min, de manière davantage préférée en moins de 100 sec,
l'extraction et la mesure des contenus de celle-ci, une division en fonction d'un résultat de mesure pour obtenir un dosage prédéterminé ; et
après la division, une stérilisation avec une stérilisation par irradiation ou une stérilisation avec l'époxyéthane, de préférence la stérilisation par irradiation.

7. Procédé, selon la revendication 6, dans lequel la membrane pour médicament stérile et à dissolution instantanée est utilisée directement dans un test de sensibilité de cellules tumorales à un médicament, ou la membrane pour médicament stérile et à dissolution instantanée est dissoute et ensuite activée en ajoutant une enzyme métabolique de médicament, pour être utilisée dans le test de sensibilité de cellules tumorales à un médicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 93111551
- CN 03102260X
- CN 102018686 A
- CN 201195724 Y

### Non-patent literature cited in the description

- **APOORVA MAHAJAN.** Formulation & Evaluation of Fast dissolving Buccal films of Sertraline. *International Journal of Drug Development & Research,* 01 March 2012, 220-226, http://www.ijddr.in/drug-development/formulation-evaluation-of-fast-dissolving-buccal-films-ofsertraline.pdf
- **NISHI THAKUR et al.** Overview ''A Novel Approach of Fast Dissolving Films and Their Patients. *Advances in Biological Research,* 01 January 2013, 50-58, http://idosi.org/abr/7(2)13/4.pdf
- **ZHANG, QIQING et al.** The study of Controlled Release Dosage Form of Antineoplastic VCR. *PROCEEDINGS OF 4TH DOCTOR FORUM ON NEW MEDICINE IN CHINA,* 1999, 262-268